# EUROPEAN PATENT APPLICATION

(11) **EP 2 314 296 A1**
(43) Date of publication of application: **27.04.2011**
(21) Application number: 09173726.2
(22) Date of filing: 22.10.2009
(51) Int. Cl.: A61K 31/4402, A61K 9/20

(54) **Orally Disintegrating Tablets of Betahistine**

(71) Applicant: Abdi Ibrahim Ilac Sanayi Ve Ticaret Anonim Sirketi, 34555 Istanbul (TR)
(72) Inventor: Farshi, Farhad, 34555, ISTANBUL (TR); Kandemir, Levent, 34555, ISTANBUL (TR); Ozge Samuk, Zerrin, 34555, ISTANBUL (TR); Ilhan, Suna Ayse, 34555, ISTANBUL (TR); Soylemez, Serdar, 34555, ISTANBUL (TR); Koc, Fikret, 34555, ISTANBUL (TR)

(57) **Abstract**

The present invention is related to fast dissolving or fast disintegrating dosage forms of betahistine.

## Description

### Technical Field

The present invention is related to orally disintegrating tablets of betahistine characterized in that betahistine particles are coated with a coating agent.

### Background Art

EP 1803446 A (QUANTUM HI-TECH (BEIJING) RESEARCH INSTITUTE) 30.04.2004 discloses an orally disintegrating formulation, comprising a therapeutically effective amount of a pharmaceutically active ingredient and a matrix, wherein the matrix contains an amino acid and pullulan. It also embraces betahistine (page 4, line 0051).

### Disclosure of Invention

The present invention provides a fast disintegrating or fast dissolving dosage forms of betahistine. Fast disintegrating dosage forms include an effective amount of betahistine or a pharmaceutically acceptable salt thereof and at least one pharmaceutically acceptable excipient.

Fast-dissolving or fast-disintegrating tablets often use the advantages of excipients which dissolve or disintegrate very fast to immediately in the salvia. These excipients can be more hygroscopic than others used for orally administered dosage forms. If the active is also hygroscopic like in the case of betahistine diHCl many problems have to be challenged to reach a non problematic and stabile product.

According to this invention, In fast disintegrating dosage forms, pharmaceutical formulations are disintegrated in less than three minutes in oral cavity. More preferably, the pharmaceutical formulations are disintegrated in oral cavity in less than two minutes and most preferably the pharmaceutical formulations are disintegrated in oral cavity in less than one minute.

Fast-dissolving or fast-disintegrating tablets are useful for elderly people, children and patients who have difficulties in swallowing tablets. Such peoples are unwilling and/or unable to swallow tablets, capsules or other traditional solid dosage forms. This is especially the case of pharmaceuticals for paediatric or geriatric use. Where patients who are not able to take it with water, fast disintegrating dosage forms would be beneficial. To solve this problem, tablets that disintegrate rapidly in the mouth were developed. Such tablets are rapidly disintegrated when in contact with saliva in the oral cavity.

According to this invention, the pharmaceutical formulation is in a dosage form can be, but not limited to, rapidly disintegrating tablets, lingual strips, sublingual tablets, lyophilized wafers, granulated particles, sublingual strips, spray and whatsoever.

A fast- disintegrating tablets may have many numerous shapes, such as dish-like, ellipsoid, rods, granules, blocks, cubes with rounded edges, or any other shape suitable for pharmaceutical administration.

According to this invention, fast disintegrating or fast dissolving dosage form includes one or more pharmaceutically acceptable excipients, carriers, or diluents/fillers. In fast disintegrating or fast dissolving formulations, surfactants, diluents, sweeteners, disintegrants, binders, lubricants, glidants, colorants, flavours, stabilizing agents, mixtures thereof and the like can be used.

Diluents/fillers are, but not limited to, mannitol, sorbitol, xylitol, microcrystalline cellulose, silicified microcrystalline cellulose , hydroxypropyl methylcellulose, hydroxypropyl cellulose , pullulan and fast dissolving carbohydrates such as Pharmaburst™, mixtures thereof and the like.

Glidants are, but not limited to, silicon dioxide, colloidal silicon dioxide, calcium silicate, magnesium silicate, magnesium trisilicate, talc, starch , mixtures thereof or whatsoever.

Binders are, but not limited to, sodium alginate,cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth,sorbitol, acacia, alginates, carrageenan, xanthan gum, locust bean gum and gum arabic, waxes, polyacrylamide, mixtures thereof, and whatsoever.

Lubricants are, but not limited to, calcium stearate, glyceryl monostearate, glyceryl behenate, glyceryl palmitostearate, hexagonal boron nitride, hydrogenated vegetable oil, light mineral oil, magnesium stearate, mineral oil, polyethylene glycol, poloxamer, sodium benzoate, sodium lauryl sulfate, sodium stearyl fumarate, stearic acid, talc, zinc stearate, mixtures thereof or whatsoever.

Disintegrants are, but not limited to, sodium starch glycolate, sodium carboxymethyl cellulose, calcium carboxymethyl cellulose, croscarmellose sodium, crospovidone, chitosan, agar, alginic acid, calcium alginate, methyl cellulose, microcrystalline cellulose, powdered cellulose, lower alkylsubstituted hydroxypropyl cellulose,hydroxylpropyl starch, low-substituted hydroxypropylcellulose, polacrilin potassium, starch, pregelatinized starch, sodium alginate, magnesium aluminum silicate, polacrilin potassium, povidone, sodium starch glycolate, mixtures thereof or whatsoever.

Disintegrant can be in mixtures or in combination with other excipient or excipients. For example Ludiflash® is made up of Kollidon® CL-SF,Kollicoat® SR30D and mannitol. Kollicoat® SR30D is made up Polyvinyl acetate stabilized with polyvinylpyrrolidone and sodium lauryl sulphate. Kollidon® CL-SF is crospovidone. Ludiflash® provides that tablets disintegrate in the mouth within seconds.

Flavors are, but not limited to, cinnamon oil,essence of apple, essence of pear, essence of peach, essence of grape, essence of strawberry, essence of raspberry, essence of cherry,essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit , vanilla, benzaldehyde , aldehyde C-8 , aldehyde C-9, aldehyde C-12 , tolyl aldehyde , mixtures thereof or whatsoever.

Sweeteners are but not limited to, corn syrup, dextrose, cyclamate, invert sugar, fructose, saccharin, aspartame, acesulfame-K, Stevia rebaudiana, sucralose, sorbitol, mannitol, zylitol, mixtures thereof and the like.

Stabilizing agents are, but not limited to, polyvinyl pyrrolidone, polymethacrylate, polyvinyl acetate phthalate, alkyl cellulose, hydroxyl alkyl cellulose, polyethylene glycol, polyethylene castor oil, polyethylene glycol sorbitan fatty acid, polyethylene polypropylene glycol, polyethylene oxide, polyoxyethylene alkyl ether, polyoxyethylene stearate, caproic acid , caprilic acid , capric acid , lauric acid , myhstic acid , palmitic acid , stearic acid , arachidic acid , behenic acid , and lignoceric acid , hydrogenated coconut fatty acids, hydrogenated palm fatty acids, hydrogenated rapeseed fatty acids, hydrogenated tallow fatty acids, and hydrogenated castor oil fatty acids, 2-ethylhexanoic acid, isostearic acid ,methanol, ethanol, n-propanol, n-butanol, n- penthanol, lauryl alcohol , myristyl alcohol , palmyl alcohol , stearyl alcohol , behenyl alcohol, isopropanol, isobutanol, sec-butanol, 2-ethylhexanol , isododecyl alcohol , isotridecyl alcohol , isostearyl alcohol , alcohol 2-octyldodecyl, glycolic acid ,lactic acid , glyceric acid , tartronic acid , malic acid , and citric acid, ethylenglycol, diethylenglycol, triethylenglycol, propylenglycol, dipropylenglycol, glycerin, diglycehn, triglycerin, sorbitol and sorbitan,sodium palmitate, sodium stearate, potassium stearate, potassium palmitate, and sodium myristate, isopropyl myristate, isopropyl palmitate, isopropyl stearate, n- butyl stearate, 2-ethylexyl palmitate, 2-ethylexyl stearate, 2-octyldodecyl myristate, 2-ethylhexyl isostearate, isostearyl isostearate, isononyl isononanoate, thethyl citrate, butyl citrate, methyl lactate, ethyl lactate, n-butyl lactate, and n-propyl lactate, diethylenglycol monoethyl ether, ethylenglycol monobutyl ether, diethylenglycol monobutyl ether, triethylenglycol monobutyl ether, thethylenglycol monoethyl, ethylenglycol monomethyl ether, diethylenglycol mono-n-propyl ether,2-octyldodecyl myhstate , isostearyl isostearate, thethyl citrate, triethyl citrate, mixtures thereof and the like.

Other ingredients such as colorants and titanium dioxide can also be used.

The fast-disintegrating or fast dissolving dosage form of betahistine diHCl is a solid dosage form that does not require water for swallowing.

Fast disintegrating or fast dissolving dosage form is typically orally disintegrating tablet that dissolves or disperses rapidly when in contact with saliva.

The compositions of the present invention can be prepared through using pharmaceutical technologies including, but not limited to, spray drying, freeze-drying, heat molding, tablet molding, sublimation, tablet and the like.

Orally disintegrating tablet can comprise sugar-based excipients,pH regulators, super-disintegrating agents , effervescent agents and further suitable excipients .

Another embodiment of the present invention provides a carbohydrate-based fast dissolving dosage form. Co-processed carbohydrates can be used in embodiments.

This invention provides taste masked orally disintegrating tablets.

In fast dissolving or fast disintegrating dosage forms one or more taste masking excipients such as flavors, sweeteners, acidic amino acids, lipids, and surfactants can further be used.

Betahistine is a relatively labile molecule due to retro Michael reaction whereby at the end dibetahistine can be formed. Therefore pH regulation in tablet, control of moisture in tablet and during processing and preventing of light is of big importance.

For pH regulation (stabilizing agent) in tablets different excipients can be use but not limited to, citric acid, lactic acid, tartaric acid, fumaric acid, malic acid, ascorbic acid, mixtures thereof and the like.

The pH in tablets is regulated to a pH value lower than 3, preferably lower than 2.9.

For determination of this value 1 g of finished product is crushed in a mortar and dispensed in 10 ml water. After stirring for 5 minutes and 1 minute sonication with ultrasound the pH value is determined by a standard pH-meter.

Preventing light from final mixture and from tablets of Betahistin at any stage of the process is due to the properties of Betahistine of great importance. If Betahistine is exposed for a longer while to light, impurities can rise dramatically to unacceptable levels. The fast disintegrating or fast dissolving betahistine diHCl finished product and/or all powders or granules to prepare such finished products have to be stored, when exposed to light for more than 1 h, in light non permeable containers (bags).

For the tabletting process the room humidity is of essential importance. At relatively high room humidity's tabletting is hardly possible and the final mixture is sticking to the tabletting punches which will disturb the continuous work flow during manufacturing step.

Packaging of orally disintegrating tablet of Betahistine is another important step due to its big influence on stability. For thus at least the secondary packaging of such tablets have to be none light permeable. The other issue is the primary packaging which has to have at least the ability to protect the product against moisture. Possible primary packaging can vary from Alu-Alu blisters to containers with desiccants or other packaging materials which can fulfill such requirements to have a stabile finished product. The fast disintegrating or fast dissolving dosage form of betahistine is packaged in tight containers with and without desicant.

A preferred embodiment is created by sugar-based fast-dissolving method, one or more sugar-based excipients can be used. These excipients are, but not limited to, amylose, dextrose, fructose, sucrose,maltose, erythritol, isomalt, lacitol, maltitol, mannitol, sorbitol, starch hydrolysate, polydextrose, glucose, xylitol, mixtures thereof and the like.

Betahistine orally disintegrating tablet comprises; betahistine or pharmaceutically acceptable salt is in the range of from about 1 % to about 40 %, binder is in the range of from about 0.1 % to about 10 %, coating agent is in the range of from about 0.1 % to about 10 %, disintegrant is in the range of from about 1 % to about 40 %, diluent/filler is in the range of from about 10 % to about 90 %, sweetener is in the range of from about 0.1 % to about 5 %, flavour is in the range of from about 0.1 % to about 5 %, glidant is in the range of from about 0.1 % to about 5 %, lubricant is in the range of from about 0.1 % to about 20 %, stabilizing agent is in the range of from about 0.1 % to about 20 % by weight. In this formulation, it is possible that one or more elements, such as stabilizing agent, may not be used. In such a case, without some elements, inventors do not aim that such formulation is excluded the invention.

An additional problem arising from oral administration .Orally disintegrating tablets that contain bitter or irritating drugs need to be taste masked in the oral cavity. Therefore a taste masking layer should be created.

The taste masking layer comprises one or more coating agents. Examples of suitable coating agents include, but not limited to, ethyl cellulose ,methyl cellulose, hydroxypropyl-methyl cellulose, hydroxyethyl cellulose, hydroxypropyl cellulose,polyvinylalcohol, polyvinyl acetate , cellulose acetate ,cellulose acetate phthalate, methylcellulose phthalate, hydroxymethyl cellulose phthalate, hydroxypropylmethylcellulose phthalate, hydroxypropylmethylcellulose succinate, cellulose acetate butyrate, methacrylate copolymers ,polymethacrylates, acetyltributyl citrate, erythritol, glyceryl palmitostearate, tributyl citrate, triethyl citrate, glyceryl monostearate, glycerin monostearate, gelatin, hydrogenated soybean oil, glyceryl monostearate,Hydrogenated oil, carboxymethyl cellulose,sodium carboxymethyl cellulose, sodium carboxymethyl starch, crosscarmellose sodium, aminoalkyl methacrylate copolymer E, low-substituted hydroxypropyl ether of cellulose, polyvinyl pyrollidone,polyethylene glycol,cellulose trimellitate,hydroxymethyl cellulose acetate succinate, resins like shellac, methylmethacrylate-methacrylic acid , methacrylic acid-ethyl acrylate,zein, hydroxyethyl-ethylcellulose phthalate, cellulose aceto succinate, polylactic acid,polyglycolic acid, gelatinized starch, ammonium hydroxide,sodium hydroxide, potassium hydroxide, magnesium hydroxide, calcium hydroxide, gums, liposomes, cyclodextrins, chitosan, gliadin,hordein,prolamine,sucrose, sorbitol, glycerin,triethylamine, diethylamine, trimethylamine, methylamine, dimethylamine, isopropylamine, triethanolamine, diethanolamine, disodium monohydrogen phosphate, dipotassium monohydrogen phosphate, mixtures thereof and the like .

The taste masking layer can further include additional excipient or excipients such as, but not limited to, pore former.

Pore formers are, but not limited to, polyethylene glycol, camphor, polyvinylpyrrolidone ,dextrose,mannose, fructose,sucrose, glucodifructose, calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, mixtures thereof and the like.

The taste-masking layer (as described herein) can be applied to the betahistine particles by any suitable method, for example fluidized bed coating methods.

Taste masking coating solution is prepared as following manner;

**Table 1**

| Ingredients | % (w/w) |
|---|---|
| Ethyl cellulose | 5 |
| Alcohol | 95 |
| **Total** | 100 |

Taste masking coating solution comprising ; (i) an inorganic acid or mixtures of inorganic acids or, (ii) an organic acid or mixtures of organic acids or, (iii) mixtures of organic acids and inorganic acids or, (iv) mixture of an organic acid and an inorganic acid or (v) an organic solvent or mixtures of organic solvents or (vi) mixtures of one or more organic solvents and any alternative (i) to (v) or (vii) de-ionized water and any alternative (i) to (v) or (viii) de-ionized water and (ix) a coating agent or mixtures of coating agents (x) optionally and additionally an excipient or mixtures of excipients.

Taste masking coating solution can include further excipients .

Organic solvents, used in preparation of solution are ,but not limited to, alcohols such as methanol, ethanol, propyl alcohol, isopropyl alcohol, 2-methyl-1-propanol etc. ;ketones such as acetone, methyl ethyl ketone, methyl isobutyl ketone etc. ; esters such as methyl acetate, ethyl acetate, propyl acetate, isopropyl acetate, ethyl formate etc. ; hydrocarbons such as heptane ; halogenated hydrocarbons such as dichloromethane.

Preferable examples of organic acids are formic acid, acetic acid, fumaric acid,tartaric acid, maleic acid, citric acid, succinic acid, malic acid, methanesulfonic acid, p-toluenesulfonic acid.

Preferable example of inorganic acid is hydrochloric acid which is in the form of solution in water.

Another aim of this invention is to provide minimum disintegrating time in oral cavity. In this point, tablet hardness is very important. According to this invention tablet hardness is in the range of 25 to 65 N. If it is desired that betahistine orally disintegrating tablet is disintegrated in 30 seconds tablet hardness should be in the range of 25 to 65 N.

This invention also provides a preparation method (Figure 1) which has following steps : a) Betahistine or pharmaceutically acceptable salt such as diHCl is mixed with a glidant or glidants and sieved. b)Sieved material obtained from step a is mixed with a diluent/filler or diluents/fillers, c)Coating agent is dissolved in alcohol, d)the powder mixture obtained from step b is coated with coating agent, e) the granules obtained from d are dried, f) Granules obtained from step e are sieved ,g) Remaining diluent, coating agent and disintegrant are added to the granules obtained from step f, h) Sweetener and flavour are added to mixture obtained from g, i)a part of powder mixture obtained from h is taken and remaining glidant is added to said powder mixture , mixed and sieved together j)obtained mixture from step i is added to mixture in step g and mixed together, k)sieved lubricant is added to the mixture obtained from h, m) final mixture is tabletted with hardness range 25-65 N.

Orally disintegrating tablet of betahistine can also be prepared by direct compression.

In direct compression formulations, betahistine particles are not coated.

If direct compression method is used, direct compression agents are, but not limited to, pregelatinised starches, polyvinylpyrrolidone, methylcellulose, microcrystalline cellulose, sucrose, lactose, dextrose, sorbitol, mannitol, lactitol, xylitol, modified calcium salt, granulated corn starch, modified rice starch, compressible sugars such as Destab™, dextrates such as Emdex ® dicalcium phosphate, hydroxypropylcellulose , methylcellulose , hydroxypropylmethylcellulose, polyethylene glycol, amylose, anhydrous calcium hydrogen phosphate, calcium sulphate,tribasic calcium phosphate, dibasic calcium phosphate,low-crystallinity powdered cellulose, silicified microcrystalline cellulose,chitin,chitosan hydrochloride, copovidone, croscarmellose sodium,dextrose, anhydrous lactose, anhydrous alpha lactose,anhydrous beta lactose, agglomerated lactose, spray-dried lactose, maltodextrin, mixtures thereof and the like. Mixtures and/or co-processed diuents which are suitable for direct compression can also be used such as anhydrous lactose- anhydrous lactitol, calcium sulphatemicrocrystalline cellulose, lactose-cellulose, lactose-starch, lactosepovidone, sucrose-maltodextrin coprecipitate and the like.

In direct compression betahistine orally disintegrating tablet comprises; betahistine or pharmaceutically acceptable salt thereof is in the range of from about 1 % to about 40 %, binder is in the range of from about 0.1 % to about 10 %, disintegrant is in the range of from about 1 % to about 40 %, direct compression agent is in the range of from about 10 % to about 90 %, sweetener is in the range of from about 0.1 % to about 5 %, flavour is in the range of from about 0.1 % to about 5 %, glidant is in the range of from about 0.1 % to about 5 %, lubricant is in the range of from about 0.1 % to about 20 %, stabilizing agent is in the range of from about 0.1 % to about 20 % by weight.

Direct compression methods provide a preparation method which has following steps :

a)Betahistine diHCl is mixed with glidant and sieved , b)direct compression agent, disintegrant, lubricant and binder, is added to mixture obtained from step a and mixed, c)Sweetener, stabilizing agent and flavour are sieved and added to mixture obtained from step b and mixed d) some of this mixture is taken and add it onto the remaining glidant and sieved and mixed . Then this mixture is mixed with mixture of step c, e)This mixture is added to mixture obtained from step d, f)sieved lubricant is added to the mixture obtained from step e, g) final powder mixture obtained from step f is blended , h) the prepared powder mixture is compressed as tablet.

In another aspect direct compression method also provide a preparation method which has following steps : a) Betahistine diHCl is mixed with glidant and sieved , b)Then, direct compression agent, disintegrant , diluent and binder are added to mixture obtained from step a and mixed together, c) sweetener and flavour are sieved and then added to mixture obtained from step b an then mixed together, d) some of the mixture obtained from step c is taken and remaining glidant is added, sieved and mixed. Then this mixture is added to mixture obtained from step c, e) sieved lubricant is added to the mixture obtained from step d, f) the final powder mixture obtained from step e is blended , g) the prepared final powder mixture obtained from step f is compressed as tablet.

Yet another aspect of this invention, direct compression provide further method which has following steps: a)Betahistine or pharmaceutically acceptable salt thereof preferanly diHCl is mixed with glidant and sieved ,b) direct compression agent, disintegrant, diluent are added to mixture obtained from step a and mixed together, c) sweetener, flavour and stabilizing agent are sieved and then added to mixture obtained from step b and mixed together, d) Sieved lubricant is added to the mixture obtained from step c, e) the final powder mixture obtained from step d is blended , f)the powder mixture obtained from step e is compressed as tablet.

This invention also provides a sieving method for betahistine orally disintegrating tablet. Accordingly, betahistine is treated with a suitable excipient or excipients and then sieved all together. Betahistine alone can not be sieved, since it adheres to sieve and bedaubs the sieve.

Another aspect of this invention is to provide suitable particle size for the mixture of sieved betahistine and a suitable excipient or excipients. The mixture obtained from sieving of betahistine and a suitable excipient or excipients have a value of D₉₀ less than 750µ. More preferably said mixture have a value of D₉₀ less than 250µ. Most preferably said mixture have value of D₉₀ less than 250µ and D₅₀ less than 80µ. Particle size is determined by using Malvern Mastersizer 2000, version 5.54.

This invention also provides dry granulation method to prepare dosage forms which involves the use of forming compacts/slugs . According to dry granulation method in the scope of this invention preparation method includes following steps ; a) betahistine or pharmaceutically acceptable salt thereof preferably diHCl, diluent, binder and lubricant are mixed , b)the mixture obtained from step a is compressed by tablet press or roller compactor, c)compressed embodiment obtained from step b is comminuted, d) the embodiment obtained from step c is sieved, e)glidant and/or lubricant is added to mixture obtained from step d and mixed, f) the mixture obtained from step e is tabletted .

Another aspect of this invention is that orally disintegrating tablets of betahistine are prepared by using melt granulation. In the scope of this invention preparation method includes following steps ; a) forming a mixture of betahistine or pharmaceutically acceptable salt thereof preferably diHCl with at least one melt component; (b) heating said mixture to a temperature substantially near the melting range of said melt component; (c) optionally, granulating said mixture in high shear or in fluid bed or any other suitable equipment.

In melt granulation, betahistine orally disintegrating tablet comprises; betahistine or pharmaceutically acceptable salt thereof preferably diHCl is in the range of from about 1 % to about 40 %, melting agent is in the range of from about 0.1 % to about 30 %, disintegrant is in the range of from about 1 % to about 40 %, flavour is in the range of from about 0.1 % to about 5 %, glidant is in the range of from about 0.1 % to about 20 % by weight, diluent is in the range of from about 5 % to about 90 % by weight.

**Example 1 (Formulation-Prepared by Wet Granulation)**

**Table 2**

| **Ingredients** | **% by weight** |
|---|---|
| Betahistine diHCl | 6,86 |
| Glidant | 0,07 |
| Diluent | 65,1 |
| Colourant | 0,20 |
| Coating Agent | 1,78 |
| Binder | 4,57 |
| Sweetener | 1,03 |
| Disintegrant | 17,14 |
| Flavour | 1,03 |
| Adsorban/Glidant | 1,07 |
| Lubricant | 1,15 |
| **Total** | **100,00** |

**Example 2 (Formulation-Prepared by Direct Compression)**

**Table 3**

| **Ingredients** | **% by weight** |
|---|---|
| Betahistine diHCl | 6,86 |
| Direct Compression Agent | 67,39 |
| Binder | 4,57 |
| Sweetener | 1,03 |
| Disintegrant | 17,14 |
| Flavour | 0,70 |
| Glidant | 0,80 |
| Lubricant | 1,51 |
| **Total** | **100,00** |

**Example 3 (Formulation-Prepared by Direct Compression)**

**Table 4**

| **Ingredients** | **% by weight** |
|---|---|
| Betahistine diHCl | 6,72 |
| Direct Compression Agent | 67,43 |
| Binder | 4,57 |
| Sweetener | 0,69 |
| Disintegrant | 16,14 |
| Flavour | 0,46 |
| Stabilizing Agent | 1,71 |
| Glidant | 1,14 |
| Lubricant | 1,14 |
| **Total** | **100,00** |

**Example 4 (Formulation-Prepared by Direct Compression)**

**Table 5**

| **Ingredients** | **% by weight** |
|---|---|
| Betahistine diHCl | 4,57 |
| Direct Compression Agent | 59,14 |
| Diluent | 20,00 |
| Disintegrant | 7,14 |
| Sweetener | 2,86 |
| Flavour | 1,43 |
| Stabilizing Agent | 1,71 |
| Lubricant | 2,29 |
| Glidant | 0,86 |
| **Total** | **100,00** |

**Example 5 (Formulation-Prepared by Melt Granulation)**

**Table 6**

| **Ingredients** | **% by weight** |
|---|---|
| Betahistine diHCl | 4,57 |
| Melting Agent | 3,43 |
| Diluent | 62,57 |
| Disintegrant | 17,14 |
| Flavour | 1,14 |
| Glidant | 11,15 |
| **Total** | **100,00** |

## Claims

1. A fast disintegrating or fast dissolving dosage form of betahistine, comprising: - an effective amount of betahistine or pharmaceutically acceptable salts thereof and - at least one pharmaceutically acceptable excipient **characterized in that** betahistine particles are coated with a coating agent or mixtures of coating agents .

2. According to claim 1, coating agent is selected from the group consisting of ethyl cellulose ,methyl cellulose, hydroxypropyl-methyl cellulose, hydroxyethyl cellulose, hydroxypropyl cellulose,polyvinylalcohol, polyvinyl acetate , cellulose acetate , cellulose acetate phthalate, methylcellulose phthalate, hydroxymethyl cellulose phthalate, hydroxypropylmethylcellulose phthalate, hydroxypropylmethylcellulose succinate, cellulose acetate butyrate, methacrylate copolymers , polymethacrylates, acetyltributyl citrate, erythritol, glyceryl palmitostearate, tributyl citrate, triethyl citrate, glyceryl monostearate, glycerin monostearate, gelatin, hydrogenated soybean oil, glyceryl monostearate,Hydrogenated oil, carboxymethyl cellulose,sodium carboxymethyl cellulose, sodium carboxymethyl starch, crosscarmellose sodium, aminoalkyl methacrylate copolymer E, low-substituted hydroxypropyl ether of cellulose,polyvinyl pyrollidone, polyethylene glycol, cellulose trimellitate,hydroxymethyl cellulose acetate succinate, resins like shellac, methylmethacrylate-methacrylic acid , methacrylic acid-ethyl acrylate,zein, hydroxyethyl-ethylcellulose phthalate, cellulose aceto succinate, polylactic acid,polyglycolic acid, gelatinized starch, ammonium hydroxide,sodium hydroxide, potassium hydroxide, magnesium hydroxide, calcium hydroxide, gums, liposomes, cyclodextrins, chitosan, gliadin,hordein,prolamine,sucrose, sorbitol, glycerin,triethylamine, diethylamine, trimethylamine, methylamine, dimethylamine, isopropylamine, triethanolamine, diethanolamine, disodium monohydrogen phosphate, dipotassium monohydrogen phosphate, mixtures thereof.

3. A fast disintegrating or fast dissolving betahistine dosage form comprising; betahistine or its pharmaceutically acceptable salts is in the range of from about 1 % to about 40 %, binder is in the range of from about 0.1 % to about 10 %, coating agent is in the range of from about 0.1 % to about 10 %, disintegrant is in the range of from about 1 % to about 40 %, diluent/filler is in the range of from about 10 % to about 90 %, sweetener is in the range of from about 0.1 % to about 5 %, flavour is in the range of from about 0.1 % to about 5 %, glidant is in the range of from about 0.1 % to about 5 %, lubricant is in the range of from about 0.1 % to about 20 %, stabilizing agent is in the range of from about 0.1 % to about 20 % by weight.

4. Taste masking coating solution for the fast disintegrating or fast dissolving betahistine or its pharmaceutically acceptable salts dosage form comprising ; (i) an inorganic acid or mixtures of inorganic acids or, (ii) an organic acid or mixtures of organic acids or, (iii) mixtures of organic acids and inorganic acids or, (iv) mixture of an organic acid and an inorganic acid or (v) an organic solvent or mixtures of organic solvents or (vi) mixtures of one or more organic solvents and any alternative (i) to (v) or (vii) de-ionized water and any alternative (i) to (v) or (viii) de-ionized water and (ix) a coating agent or mixtures of coating agents (x) optionally an excipient or mixtures of excipients.

5. A preparation method of fast disintegrating or fast dissolving betahistine or its pharmaceutically acceptable salts dosage form **characterized in that** said method has the following steps : a) Betahistine or its pharmaceutically acceptable salts is mixed with a glidant or glidants and sieved. b)Sieved material obtained from step a is mixed with a diluent/filler or diluents/fillers, c)Coating agent is dissolved in alcohol, d)the powder mixture obtained from step b is coated with coating agent, e) the granules obtained from d are dried, f) granules obtained from step e are sieved , g) Remaining diluent, coating agent and disintegrant are added to the granules obtained from step f, h) sweetener and flavour are added to mixture obtained from g, i)a part of powder mixture obtained from f is taken and remaining glidant is added to said powder mixture ,mixed and sieved together j)obtained mixture from step i is added to mixture in step g and mixed together, k)sieved lubricant is added to the mixture obtained from h, j) final mixture is tabletted with hardness range 25-65 N.

6. A fast disintegrating or fast dissolving dosage form of betahistine or its pharmaceutically acceptable salts **characterized in that** dosage form is prepared by direct compression.

7. A fast disintegrating or fast dissolving dosage form of betahistine or its pharmaceutically acceptable salts comprising: - an effective amount of betahistine or a pharmaceutically acceptable salt thereof and - at least one direct compression agent

8. According to claim 6 direct compression agent is selected from the group consisting of pregelatinised starches, polyvinylpyrrolidone, methylcellulose, microcrystalline cellulose, sucrose, lactose, dextrose, sorbitol, mannitol, lactitol, xylitol, modified calcium salt, granulated corn starch, modified rice starch, compressible sugars such as Destab™, dextrates such as Emdex ® dicalcium phosphate, hydroxypropylcellulose , methylcellulose , hydroxypropylmethylcellulose, polyethylene glycol, amylose, anhydrous calcium hydrogen phosphate, calcium sulphate, tribasic calcium phosphate, dibasic calcium phosphate,low-crystallinity powdered cellulose, silicified microcrystalline cellulose,chitin,chitosan hydrochloride, copovidone, croscarmellose sodium,dextrose, anhydrous lactose, anhydrous alpha lactose,anhydrous beta lactose, agglomerated lactose, spray-dried lactose, maltodextrin, mixtures thereof and the like. Mixtures and/or co-processed diuents which are suitable for direct compression can also be used such as anhydrous lactose- anhydrous lactitol, calcium sulphate,microcrystalline cellulose, lactose-cellulose, lactose-starch, lactosepovidone, sucrose-maltodextrin coprecipitate or mixtures thereof.

9. A preparation method of fast disintegrating or fast dissolving betahistine or its pharmaceutically acceptable salts dosage form by direct compression **characterized in that** said method has the following steps : a)Betahistine is mixed with glidant and sieved , b)direct compression agent, disintegrant, lubricant and binder, is added to mixture obtained from step a and mixed, c)Sweetener, stabilizing agent and flavour are sieved and added to mixture obtained from step b and mixed d) some of this mixture is taken and added it onto the remaining glidant sieved and mixed . Then this mixture is mixed with mixture of step c, e)This mixture is added to mixture obtained from step d, f)sieved lubricant is added to the mixture obtained from step e, g) final powder mixture obtained from step f is blended, h) the prepared powder mixture is compressed as tablets

10. A preparation method of fast disintegrating or fast dissolving betahistine or its pharmaceutically acceptable salts dosage form by direct compression **characterized in that** said method has the following steps : a) Betahistine is mixed with glidant and sieved , b)Direct compression agent, disintegrant , diluent and binder are added to mixture obtained from step a and mixed together, c) sweetener and flavour are sieved then added to mixture obtained from step b and mixed together, d) some of the mixture obtained from step c is taken and remaining glidant is added, sieved and mixed. Then this mixture is added to mixture obtained from step c, e) sieved lubricant is added to the mixture obtained from step d, f) the final powder mixture obtained from step e is blended , g) the prepared final powder mixture obtained from step f is compressed as tablet.

11. A preparation method of fast disintegrating or fast dissolving betahistine or its pharmaceutically acceptable salts dosage form by direct compression **characterized in that** said method has the following steps : a)Betahistine is mixed with glidant and sieved , b) direct compression agent, disintegrant, diluent are added to mixture obtained from step a and mixed together, c) sweetener, flavour and stabilizing agent are sieved then added to mixture obtained from step b and mixed together, d) Sieved lubricant is added to the mixture obtained from step c, e) the final powder mixture obtained from step d is blended , f) the powder mixture obtained from step e is compressed as tablet.

12. A fast disintegrating or fast dissolving betahistine or its pharmaceutically acceptable salts dosage form prepared by direct compression **characterized in that** betahistine or its pharmaceutically acceptable salt is in the range of from about 1 % to about 40 %, binder is in the range of from about 0.1 % to about 10 %, disintegrant is in the range of from about 1 % to about 40 %, direct compression agent is in the range of from about 10 % to about 90 %, sweetener is in the range of from about 0.1 % to about 5 %, flavour is in the range of from about 0.1 % to about 5 %, glidant is in the range of from about 0.1 % to about 5 %, lubricant is in the range of from about 0.1% to about 20 %, stabilizing agent is in the range of from about 0.1 % to about 20 % by weight.

13. A fast disintegrating or fast dissolving dosage form of betahistine or its pharmaceutically acceptable salts **characterized in that** dosage form is prepared by dry granulation.

14. A preparation method of fast disintegrating or fast dissolving betahistine or its pharmaceutically acceptable salts dosage form by dry granulation **characterized in that** said method has the following steps : a. betahistine, diluent, binder and lubricant are mixed , b)the mixture obtained from step a is compressed by tablet press or roller compactor, c)compressed embodiment obtained from step b is comminuted, d) the embodiment obtained from step c is sieved, e)glidant and/or lubricant is added to mixture obtained from step d and mixed, f) the mixture obtained from step e is tabletted.

15. A fast disintegrating or fast dissolving dosage form of betahistine or pharmaceutically acceptable salt thereof **characterized in that** dosage form is prepared by melt granulation.

16. A melt granulation method to prepare fast disintegrating or fast dissolving dosage form of betahistine or pharmaceutically acceptable salt thereof includes following steps; a) forming a mixture of betahistine with at least one melt component; (b) heating said mixture to a temperature substantially near the melting range of said melt component; (c) optionally, granulating said mixture in high shear or in fluid bed or any other suitable equipment.

17. A fast disintegrating or fast dissolving dosage form of betahistine or pharmaceutically acceptable salt thereof which is prepared by melt granulation **characterized in that** it comprises; betahistine is in the range of from about 1 % to about 40 %, melting agent is in the range of from about 0.1 % to about 30 %, disintegrant is in the range of from about 1 % to about 40 %, flavour is in the range of from about 0.1 % to about 5 %, glidant is in the range of from about 0.1 % to about 20 % by weight, diluent is in the range of from about 5 % to about 90 % by weight.

18. A sieving method for fast disintegrating or fast dissolving betahistine or pharmaceutically acceptable salt thereof dosage form **characterized in that** betahistine is treated with a suitable excipient or excipients and then sieved all together.

19. A fast disintegrating or fast dissolving betahistine or pharmaceutically acceptable salt thereof dosage form wherein particle size D₉₀ of sieved betahistine and a suitable excipient or excipients is not greater than 750µ.

20. According to claim 19, more preferably, particle size D₉₀ of sieved betahistine and a suitable excipient or excipients is not greater than 250µ.

21. According to claim 20, most preferably particle size D₉₀ of sieved betahistine and a suitable excipient or excipients is not greater than 250µ and particle size D₅₀ of sieved betahistine and a suitable excipient or excipients is not greater than 80µ.

22. A fast disintegrating or fast dissolving dosage form of betahistine or pharmaceutically acceptable salt thereof **characterized in that** the fast disintegrating or fast dissolving dosage form of betahistine has a pH value of lower than 3 if 1 g of tablet is suspended in 10 mL water.

23. According to claim 22 the fast disintegrating or fast dissolving dosage form of betahistine has a pH value of lower than 2,9 if 1 g of tablet is suspended in 10 mL water.

24. A fast disintegrating or fast dissolving dosage form of betahistine or pharmaceutically acceptable salt thereof **characterized in that** betahistine finished product and/or all powders or granules to prepare such finished product are stored, when exposed to light for more than 1 h, in light non permeable containers (bags).

25. A fast disintegrating or fast dissolving dosage form of betahistine or pharmaceutically acceptable salt thereof **characterized in that** the fast disintegrating or fast dissolving dosage form of betahistine has a secondary packaging which is at least non-light permeable.

26. A fast disintegrating or fast dissolving dosage form of betahistine or pharmaceutically acceptable salt thereof **characterized in that** the fast disintegrating or fast dissolving dosage form of betahistine has a primary packaging which protects the product against moisture.

27. A fast disintegrating or fast dissolving dosage form of betahistine or pharmaceutically acceptable salt thereof **characterized in that** the fast disintegrating or fast dissolving dosage form of betahistine is packaged in Alu-Alu blisters.

28. A fast disintegrating or fast dissolving dosage form of betahistine or pharmaceutically acceptable salt thereof **characterized in that** the fast disintegrating or fast dissolving dosage form of betahistine is packaged in tight containers with or without desiccant.

29. A fast disintegrating or fast dissolving dosage form of betahistine or pharmaceutically acceptable salt thereof **characterized in that** dosage form is prepared by wet granulation.

30. According to preceding claims, the fast disintegrating or fast dissolving dosage form of betahistine or pharmaceutically acceptable salt thereof is disintegrated in oral cavity in less than three minutes.

31. According to claim 30, the fast disintegrating or fast dissolving dosage form of betahistine is disintegrated in oral cavity in less than two minutes.

32. According to claim 31, the fast disintegrating or fast dissolving dosage form of betahistine is disintegrated in oral cavity in less than one minutes.

33. According to claim 32, the fast disintegrating or fast dissolving dosage form of betahistine is disintegrated in oral cavity in less than 30 seconds.

34. According to preceding claims, the fast disintegrating or fast dissolving dosage form is orally disintegrating tablet.
